(19) Europäisches Patentamt / European Patent Office / Office européen des brevets

(11) **EP 4 389 111 A1**

(12) **EUROPEAN PATENT APPLICATION**

(43) Date of publication:
**26.06.2024 Bulletin 2024/26**

(21) Application number: **22461654.0**

(22) Date of filing: **23.12.2022**

(51) International Patent Classification (IPC):
**A61K 8/9789** (2017.01)    **A61Q 19/08** (2006.01)
**A61K 36/28** (2006.01)    **A61K 36/41** (2006.01)

(52) Cooperative Patent Classification (CPC):
(C-Sets available)
**A61K 8/9789; A61K 36/28; A61K 36/41;**
**A61Q 19/08;** A61K 2236/00; A61K 2800/522;
A61K 2800/5922    (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Aero-Bw K. Domagala Sp. Jawna**
**32-500 Chrzanów (PL)**

(72) Inventors:
• **Domagala, Dominik**
  **30-347 Kraków (PL)**
• **Domagala, Barbara**
  **30-347 Kraków (PL)**

(74) Representative: **JD&P Patent Attorneys**
**Joanna Dargiewicz & Partners**
**ul. Mysliborska 93A/50**
**03-185 Warszawa (PL)**

(54) **METHOD OF PREPARATION OF COMPOSITION CONTAINING PLANT METABOLITE EXTRACT AND COMPOSITION CONTAINING PLANT METABOLITE EXTRACT**

(57)    The subject matter of the invention is a method of preparing composition containing plant metabolite extract, which includes grinding of plant material; maceration of the ground plant material with distilled water, wherein the weight ratio of plant material to distilled water is 1: 20 per dry plant material; extraction of the plant material previously subjected to maceration using ultrasonic extraction and stirring; separation of the extract from the plant material using filtration; concentration of the obtained plant metabolite extract; adding and stirring the remaining components of the composition including water, glycerin, citric acid, sodium benzoate, gluconolactone, and calcium gluconate, to obtain a finished composition. Subject matter of the invention is also a composition containing plant metabolite extract.

Fig. 1

(52) Cooperative Patent Classification (CPC): (Cont.)

C-Sets
**A61K 36/28, A61K 2300/00;**
**A61K 36/41, A61K 2300/00**

**Description**

The subject matter of the present invention is a method of preparing composition containing plant metabolite extract and a composition containing plant metabolite extract, in particular from *Kalanchoe pinnata* and *Calendula officinalis.*

The purpose of the present invention was to develop a method of preparing composition containing plant metabolite extract by extracting the active ingredients in the least altered form possible (without destroying the phytochemicals). Studies were carried out on *Kalanchoe pinnata* and *Calendula officinalis.*

## *Kalanchoe pinnata*

*Kalanchoe pinnata,* the air plant, is a plant well-known in temperate and tropical countries, used to treat a wide range of conditions. Among them are abscesses, tonsil infections, arthritis, mycosis, psoriasis, or boils.

To date, numerous phytochemicals such as alkaloids, flavonoids, phenolic compounds, tannins, macronutrients, micronutrients have been detected in the air plant.

Kaempferol 3-$O$-$\alpha$-L-arabinopyranosyl-(1→)-$\alpha$-L-rhamnopyranoside, quercetin 3-$O$-$\alpha$-L-arabinopyranosyl-(1→2)-$\alpha$-L-rhamnopyranoside, 4',5-dihydroxy-3',8-dimethoxyflavone 7-$O$-$\beta$-D-glucopyranoside and quercetin are responsible for antileishmanial activity.

From fresh leaves of *Kalanchoe pinnata* were isolated i.a. bryophyllol, bryophollone, bryophollenone, bryophynol, 18$\alpha$-oleanane, $\psi$-taraxastero), $\beta$-amyrin acetate, a mixture of $\alpha$- and $\beta$-amyrins and their acetates. Two insecticidal bufadienolides identified as bryophyllin A and bryophyllin C were isolated by methanol extraction. Five bufadienolides, such as bryophollone, bryophyllin A, bryophyllin C, bersaldegenin 3-acetate, bersaldegenin 1,3,5-orthoacetate and daigremontianin were also isolated.

In addition, the presence of 1-octane-3-$O$-$\alpha$-L-arabinopyranosyl-(1→6)-glucopyranoside was determined as well as the content of cardienolide and steroids including $\beta$-sitosterol, bryophyllol, bryophynol, bryotoxin A, bryotoxin B, campesterol, 24-ethyl-25-hydroxycholesterol, isofucosterol, clionasterol, codisterol, peposterol, 22-dihydrobrassicasterol, clerosterol, 24-epiclerosterol, 24-ethyldesmosterol, stigmasterol, which were isolated from the above-ground parts.

The leaves of the plant in question contain amino acids, casein hydrolysate, nicotinamide, carbohydrates, proteins, lipids, minerals; sodium, calcium, potassium, phosphorus, magnesium, iron, copper, zinc, and sugars.

Many enzymes were also detected, i.a. phosphoenolpyruvate carboxykinase (PCK), phosphoenolpyruvate carboxylase (PEPC), pyruvate orthophosphate dikinase (PPDK), ribulose-1,5-bisphosphate carboxylase/oxygenase.

Protein compounds that play a major role in metabolism such as phosphoglycerate kinase, carbonic anhydrase, glycolate oxidase, fructose-bisphosphate aldolase, DNA topoisomerases have also been discovered.

In terms of pharmacological activity, *Kalanchoe pinnata* shows antitumor, anticonvulsant, antidiabetic, antimycotic, antileishmanial (LMA), and antibacterial activities. It also has antinociceptive and anti-inflammatory effects, shows antiproliferative, anti-ulcer, diuretic and anti-urolithic activities. It shows hepatoprotective activity, immunomodulatory effect, renoprotective effect, neuroinflammatory and muscle relaxant activities (as uterine relaxant) and wound healing activity.

## *Calendula officinalis*

*Calendula officinalis* or common marigold is traditionally used to treat internal organ inflammations, gastrointestinal ulcers and dysmenorrhea, and as a diuretic and diaphoretic in convulsions. It is also used for oropharyngeal mucositis, wounds, and burns.

Marigold is a cleansing and detoxifying herb, and the marigold infusion treats chronic infections. Dried flower heads have been used for their antipyretic, antitumor and scarifying properties. The medicinal properties of *C. officinalis* have been mentioned in the Ayurvedic and Unani systems of medicine, indicating that the leaves and flowers have antipyretic, anti-inflammatory, antiepileptic and antimicrobial effects. In traditional and homeopathic medicine *C. officinalis* was used for poor eyesight, irregular menstruation, varicose veins, hemorrhoids and duodenal ulcers. Of the various species in the genus *Calendula* sp., *C. officinalis* is the only one that is widely used clinically around the world. A cream containing common marigold extract was found to be effective in treatment of dextran-induced and burn edema, as well as in acute lymphedema in rats. The common marigold flower essential oil inhibited the *in vitro* growth of *Bacillus subtilis, Escherichia coli, S. aureus, Pseudomonas aeruginosa* and *Candida albicans.*

## Psoriasis

Plant metabolites, especially from *Kalanchoe pinnata* and *Calendula officinalis,* are expected to find their use in treating or alleviating the symptoms of psoriasis, a relatively common inflammatory skin disease that affects nearly 2-5% of the population. Its clinical symptoms are characterized by an acute inflammatory reaction in the first phase, progressing to a chronic form. The detailed mechanism of psoriasis is not clarified. The predominant view, pointing to a dysfunction of the cellmediated adaptive immune system, based on excessive activation of dendritic cells, Th1/Th17 cells and production of cytokines, primarily IL-17a, IL-23, TNF$\alpha$ and interferon $\gamma$.

As mentioned above, plant extracts of *Kalanchoe pinnata* and *Calendula officinalis* have been used in folk and traditional medicine for thousands of years. Modern science confirms their effects, pointing to their ability to relieve inflammation, antitumor activity, wound healing promotion, antibacterial activity, etc. Therefore, the authors of the present invention propose the use of *Kalanchoe pinnata* and *Calendula officinalis* plant extracts in supporting psoriasis therapy. The base of psoriasis is a deregulated inflammation. Despite the still unexplained mechanism of its origins, the key role play neutrophil accumulation, local production of highly pro-inflammatory cytokines (TNF$\alpha$, IL-17, IL-23) and other transmitters (leukotrienes and other arachidonic acid derivatives), and the presence of Th17 and dendritic cell populations. Because of the fundamental role of the NF$\kappa$B signaling pathway in regulating the inflammatory response, regardless of the actual mechanism, reducing the activity of this transcription factor will translate into alleviation of inflammation. In the same sense, the exemplary effect of non-steroidal anti-inflammatory drugs based on COX-2 inhibition, although directly affecting only a single aspect of the process, results in the alleviation of the entire inflammation. NF$\kappa$B is universal in nature and is found in lymphocytes as well as in epithelial cells and neutrophils, i.e. all involved in the psoriatic process, moreover, it has a fundamental function in regulating the inflammatory process in the epithelium.

**Summary of the invention**

The present invention relates to a method of preparing composition containing plant metabolite extract as defined in claim 1 and a composition containing plant metabolite extract as defined in claim 12. Preferred embodiments are defined in the dependent claims.

**Drawing**

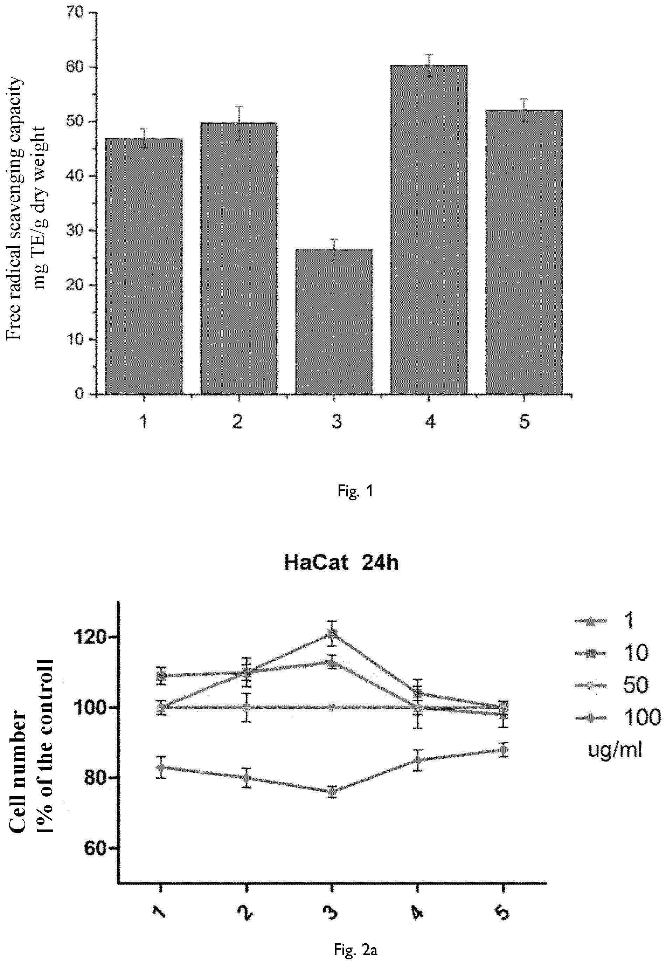

Fig. 1 shows the capacity of extracts 1-5 to scavenge free radicals in the DPPH assay. The assay results are expressed in trolox equivalents (TEs) (mg TE/g dry weight). This unit expresses the DPPH radical quenching capacity of 1 g dry weight of the tested extract equivalent to the DPPH radical quenching capacity of 1 mg of trolox. All assays were performed in triplicate, and the results were averaged.

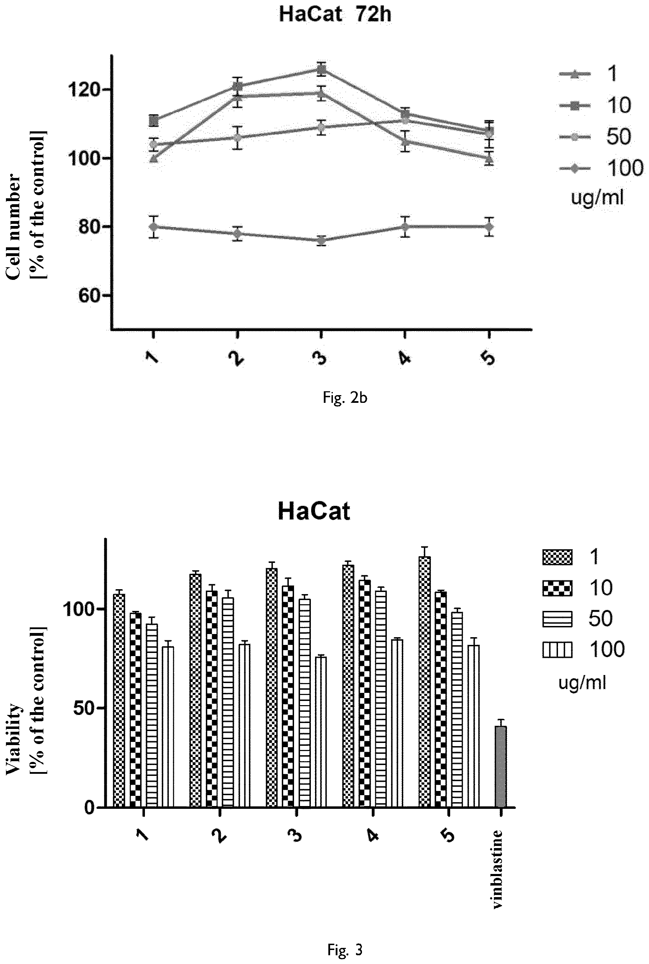

Fig. 2a shows the proliferation of keratinocytes of the HaCat line incubated in the presence of the tested extracts 1-5 for 24 hours, and Fig. 2b-for 72 hours. Cell proliferation was determined using crystal violet assay. Each experiment was performed in triplicate. The graphs show the number of live, proliferating cells expressed as a percentage of control (cells not incubated with the test compounds) $\pm$ SEM.

Fig. 3 shows the viability of keratinocytes of the HaCat line incubated in the presence of tested extracts 1-5 for 24 hours. Cell viability was determined using MTT assay. Each experiment was performed in triplicate. The graphs show the number of viable cells expressed as a percentage of control (cells not treated with compounds) $\pm$ SEM.

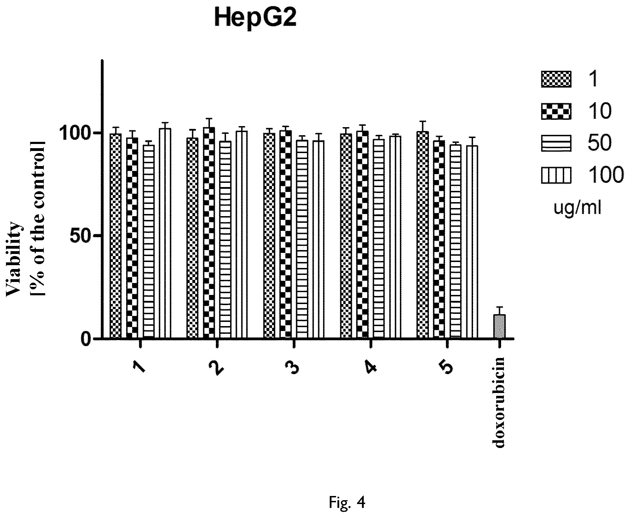

Fig. 4 shows the viability of hepatocytes of the HepG2 line incubated in the presence of tested extracts 1-5 for 24 hours. Cell viability was determined using MTT assay. Each experiment was performed in triplicate. The graphs show the number of viable cells expressed as a percentage of control (cells not treated with compounds) $\pm$ SEM.

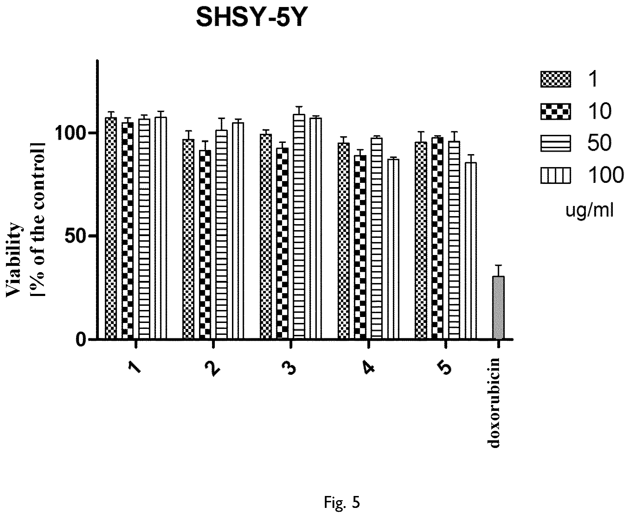

Fig. 5 shows the viability of SHSY-5Y line cells incubated in the presence of tested extracts 1-5 for 24 hours. Cell viability was determined using MTT assay. Each experiment was performed in triplicate. The graphs show the number of viable cells expressed as a percentage of control (cells not treated with compounds) $\pm$ SEM.

**EXAMPLES**

Example 1. *Kalanchoe pinnata* **culture**

From the various types of media tested during culturing, the optimal medium composition for the growth of *Kalanchoe pinnata* was selected in the form of Murashige and Skoog (MS) medium supplemented with individually selected growth regulators summarized in the table below.

Table 1: Growth regulators for optimal *Kalanchoe pinnata in vitro* culture

| Component name | Chemical formula | Concentration | |
|---|---|---|---|
| | | [mg/L] | [‰] |
| 6-Benzylaminopurine (BAP) | $C_{12}H_{11}N_5$ | 1.0 | 0.0010 |
| 1-Naphthylacetic acid (NAA) | $C_{12}H_{10}O_2$ | 0.5 | 0.0005 |

Technology of preparation of 1 L of medium when using ready-made Murashige and Skoog mixtures with the addition of **1.0 mg/L BAP and 0.5 mg/L NAA:**

1. Measure 1 L of distilled water and add the ready-made Murashige and Skoog (MS) medium according to the manufacturer's recommendations-for example, in the case of the Duchefa Biochemie used, it is 4.41 g/L.
2. Add sucrose: 30 g/L (unless it is included in the ready-made medium).
3. Add growth regulators: **1 mL BAP** and **0.5 mL NAA.**
4. Stir in a stirrer until all components dissolve.
5. Set the pH of the medium at **5.7-5.8** adjusting it towards acidity with hydrochloric acid (1M HCl), and towards alkalinity with sodium hydroxide (1M NaOH).
6. Pour the prepared medium into bottles, and then sterilize according to the procedure described below.

**Passaging**

Perform all operations **under sterile conditions**-in the laminar flow cabinet.

Before putting the sterilized items, as well as the initial bioreactor into the laminar flow cabinet, spray them with a 70% spirit solution. All items in the laminar flow cabinet except the bioreactor with the initial culture, the small sterilizer and the beaker with 70% spirit solution should be sterilized in the autoclave.

Passaging from one bioreactor to another:

- Pour the sterilized medium into the sterilized bioreactor: 0.5 L/bioreactor.

- Transfer some of the material (about 10-15 g) from the initial bioreactor to the newly established one. Perform this operation with previously sterilized tweezers, or other instruments. During transferring, separate the material with tweezers into smaller pieces or cut it on a sterilized paper with a scalpel sterilized in the small sterilizer (about 2x2 cm, so as to contain at least 3 new shoots).

- Seal the bioreactor tightly.

- Connect the newly established bioreactor to the medium immersion system. Make sure that the system is in a closed circuit-all free ends should be secured with plugs.

- Repeat the operations as needed.

- **Rinse** from plant parts the instruments used for passage in a beaker with a 70% spirit solution **before** putting them into the small sterilizer. If necessary, sterilize again in the sterilizer in the laminar flow cabinet.

The best biomass growth was obtained using a 2-minute medium immersion period every 1.5 hours using Plantform bioreactors. The described system of bioreactor flooding is used in a continuous mode, i.e. 24 hours/7 days a week.

The culture time is from 5 weeks to 6 weeks.

Lighting: continuous, white LED with an intensity of 2.75 W/m$^2$

Temperature: 25±3°C.

Example 2. *Calendula officinalis* culture

*Table 2: Growth regulators for optimal Calendula officinalis in vitro culture*

| Component name | Chemical formula | Concentration | |
|---|---|---|---|
| | | [mg/L] | [‰] |
| Indole-3-butyric acid (IBA) | $c_{12}H_{13}N0_2$ | 1 | 0.0020 |
| 6-Benzylaminopurine (BAP) | $C_{12}H_{11}N_5$ | 2 | 0.0010 |

The procedure for preparing 1 L of medium when using ready-made Murashige and Skoog mixtures is analogous to that of *Kalanchoe pinnata,* but it is necessary to pay attention to **other growth regulators,** in this case **1 IBA and 2 BAP.**

**1.1. Sterilization**

The sterilization process is identical to that for *Kalanchoe pinnata.*

**1.2. Passaging**

Perform all operations **under sterile conditions-in** the laminar flow cabinet.
Before putting the sterilized items, as well as the mother bioreactor into the laminar flow cabinet, spray them with a 70% spirit solution. All items in the laminar flow cabinet except the *mother* bioreactor with the plant, the small sterilizer and the beaker with 70% spirit solution should be sterilized in the autoclave.
Passaging from one bioreactor to another:

- Pour the sterilized medium into the sterilized bioreactor: 0.5 L/bioreactor.
- Remove some of the material from the old bioreactor and put it onto a sterilized paper. Perform this operation with previously sterilized tweezers, or other instruments. Cut the material with a scalpel sterilized in a small sterilizer (about $2\times2$ cm). **When passaging *Calendula officinalis,* it is important not to cut the shoots.**
- Transfer such prepared material to a new bioreactor.
- Repeat the operations as needed.
- Rinse from plant parts the instruments used for passage in a beaker with a 70% spirit solution before putting them into the small autoclave. If necessary, sterilize again in the sterilizer in the laminar flow cabinet.

**1.3. Culture**

The best biomass growth is obtained using a 2-minute medium immersion period, every 1.5 hours using Plantform bioreactors. The described system of bioreactor flooding is used in a continuous mode, i.e. 24 hours/7 days a week.

The culture time is 4 weeks.

Lighting: continuous, white LED with an intensity of 2.75 $W/m^2$

Temperature: 25$\pm$3°C.

Example 3. Obtaining metabolites from *Kalanchoe pinnata (Bryophyllum pinnatum)* and *Calendula officinalis*

Since there are known and unknown compounds in the mother plant, it was decided to obtain them, paying special attention to their thermal lability.
40 °C was set as the limit temperature up to which the stability of thermally labile compounds can be assumed (which is associated with the use of raw plants in the folk medicine), and this temperature was not exceeded at any stage of the process.
The aim was to develop a formulation of a finished composition containing metabolite extract of the plants under study, which can be stored, used for cosmetic-pharmaceutical formulations, as well as made available to other business entities.

Water, glycerin and the preservative complex Gluconolactone SB (Sodium Benzoate, Gluconolactone, Calcium Gluconate), which acts on a wide spectrum of Gram-positive and Gramnegative bacteria, and works best at pH 3-6, were used as raw materials to support the placement of the stem cell metabolites of the plants in question in water-oil phase systems, and therefore, the possibility of pH adjustment with citric acid was introduced.

It was determined that the composition of the finished composition without encapsulation of the active ingredients in liposomes is as follows:

*Table 3: Extract from stem cells obtained from Kalanchoe pinnata*

| Trade name | INCI name | Composition [%] |
|---|---|---|
| Water | Aqua | 49.99 |
| Glycerin | Glycerin | 26.20 |
| Air plant stem cell extract | Kalanchoe pinnata Callus Culture Extract | 22.00 |
| Citric acid | Citric Acid | 0.60 |
| Sodium benzoate | Sodium Benzoate | 0.30 |
| Gluconolactone | Gluconolactone | 0.90 |
| Calcium gluconate | Calcium Gluconate | 0.01 |

*Table 4: Extract from stem cells obtained from Calendula officinalis*

| Trade name | INCI name | Composition [%] |
|---|---|---|
| Water | Aqua | 49.99 |
| Glycerin | Glycerin | 26.20 |
| Common marigold stem cell extract | Calendula officinalis Callus Culture Extract | 22.00 |
| Citric Acid | Citric Acid | 0.60 |
| Sodium Benzoate | Sodium Benzoate | 0.30 |
| Gluconolactone | Gluconolactone | 0.90 |
| Calcium Gluconate | Calcium Gluconate | 0.01 |

Due to the diversity of culture harvests, two preparatory methods were developed for extracting material. One involving direct fresh material extraction, and the other, in the case of large harvests and low demand for the finished product, drying the material. Dry material can be kept under optimal conditions for much longer, thus reducing losses.

Preparation of dry material

Raw *Kalanchoe pinnata* plant material obtained from the culture should be rinsed with distilled water and subjected to:

- drying at 40 °C in the dryer,
  or
- lyophilization.

The length of both processes depends on the amount of mass introduced and the degree of its fineness. Both processes should be carried out until dry material is obtained.

After obtaining dry material, it should be ground in an electric grinder for 20 s.

In the case of *Calendula officinalis* plant material, which is purchased in the form of already dry material, its portions should be directly subjected to grinding in an electric grinder for 20 s.

The grinding process should lead to grinding a single particle to a size of ≤0.5 mm, which should be confirmed by sieve analysis. This process is necessary to increase the wetting surface of the extracted material.

Important!: During grinding, special attention should be paid to the temperature of the material to avoid overheating.

The weight ratio of the extracted dried material to distilled water was assumed to be 1:20. After placing the water and ground dried material in a beaker, the maceration process was carried out for 40 min, using EnviSense AM 300S-H mechanical stirrer, setting a rotation speed of 450 rpm. Maceration of the suspended plant product was carried out to thoroughly wet the ground material.

Preparation of wet material

The raw *Kalanchoe pinnata* plant material obtained from the culture should be rinsed with distilled water, divided into portions and placed in a food processor. Blend each individual portion for 15 s.

Based on repeated attempts and studies, it was discovered that statistically the dry matter content of the *Kalanchoe pinnata* suspension after blending is 3.53%. Thus, making the above assumption, it is necessary to pour such an amount of distilled water into the blended mass that the ratio of water to dry matter is 1:20. After obtaining the appropriate weight ratio of the plant and distilled water, the maceration process should be carried out for 40 min, using EnviSense AM 300S-H mechanical stirrer, setting a rotation speed of 450 rpm, so the maceration process is carried out analogously to the dry material.

40°C was set as the limit temperature up to which the stability of thermally labile compounds can be assumed (which is associated with the use of raw plants in the folk medicine), and this temperature was not exceeded at any stage of the process. The raw *Kalanchoe pinnata* plant material obtained in the culture was subjected to drying at 40°C in an oven until dried.

## *Kalanchoe pinnata*

35 g of dried material was weighed and subjected to grinding 3 times in an electric grinder for 1 minute each 15 seconds with a pause and measuring the heating temperature during grinding. If it rose above 35°C, the grinding was stopped to cool the material.

The grinding process resulted in grinding a single particle to a size of 0.4 mm, which was confirmed by sieve analysis using a 0.5 mm sieve. Such fineness was necessary so that the solvent would have the opportunity to reach as much surface area of the extracted material as possible.

The use of water as a solvent is scientifically and economically justified. Ultrasonic extraction was chosen as the extraction method, using Hielscher UP 200 St laboratory sonicator and EnviSense AM 300S-H mechanical stirrer placed in a 1000 mL beaker. The beaker was placed in a vessel with ice water cooling capability. The weight ratio of the extracted dry matter to DM water was 1:20.

Selection of ultrasonication parameters:

- Frequency not higher than 25 KHz, so as not to allow the transition of phytochemicals to their free radical form
- Amount of energy delivered: 40 W*s/mL, at a ratio of dry material: solvent 1:20
- Amplitude value of 40%, electronically adjustable (Hielscher UP 200 St)
- Pulsating sonicator duty cycle $\alpha = 4/5$ $\alpha = \tau_{ON}/(\tau_{ON} + \tau_{OFF})$

$$\tau_{ON} = \text{sonicator generator on time} = 10\ s$$

$$\tau_{OFF} = \text{sonicator generator off time} = 2\ s$$

- Sonication power value 33-36 W.

Ground dried *Kalanchoe pinnata* stem cells in the amount of 35 g were placed in a 1000 mL beaker and 700 mL of distilled water was poured over. A sonicator sonotrode was introduced into the beaker and a mechanical stirrer was placed at an angle of 65°. Then, at 450 rpm, maceration of the suspended plant product was carried out for 30 minutes to thoroughly wet the ground material. Sonication was started with continuous stirring of the suspension and continuous temperature control. If it rose to 30°C, ice was added to the cooling medium. After delivering a total energy of 28,000 W*s, the process was terminated. The plant parts were separated from the extract by vacuum filtration, using Whatman No. 1 filter paper. The filtration was repeated twice, each time washing the filter paper with 50 mL of water. 585.25 g of extract was obtained, which was then concentrated in a rotary vacuum evaporator IKA RV8 using a WELCH LVS 105T vacuum pump set to a vacuum parameter of 42 mbar. The heating temperature of the flask was set at 40°C. However, previously, 8.0102 g of extract was taken for gravimetric analysis of the dry matter determined, and evaporated to dryness at 85°C. 0.1547 g dry weight (metabolites) was obtained and thus there was 11.148 g of metabolites left in solution in the flask of the device (taking into account the amount taken for gravimetric analysis). Next, it was calculated how much water should be evaporated so that there is a system of 22% of metabolites and 49.39% of water left in the flask, characterizing the finished product described above. There should be 25.027 g of water left in the flask at the end of the process, so considering the sample taken for gravimetric analysis, 541.06 g of water should have been evaporated. This value was weighed in the flask receiving the solvent and the level to which this water content reaches in the flask receiving the distillate was marked. After pouring out the water used to determine the level, the rotary vacuum evaporator system was assembled and the process was carried out until the water was evaporated to the level determined before.

The temperature of the preheating bath was 40°C and distillation was started at a vacuum of 51 mbar.

During evaporation, the vacuum was reduced by about 1/3 to 49 mbar, and in the final stage of the solvent removal, the vacuum was set at 40 mbar. A Zhengzhou Well DLS 20L -300C refrigerator with a refrigerant temperature setting of 2°C was used for cooling.

After completing the distillation, the residue in the flask was weighed and a solution of 36.275 g of secondary metabolites dissolved in water was obtained.

The remaining components were then added according to Table 1, and after preventive filtration through a Whatman No. 1 filter paper, 49 g of finished product was obtained, which is both of a commercial nature and for introduction into cosmetic-pharmaceutical products.

Eighteen attempts were made to optimize this process, using various variable parameters.

### *Calendula officinalis*

The process described below is based on the technology of obtaining stem cell extract without liposomes and the following description is also the final result of optimization.

The process used 55.5 g of dried *Calendula officinalis* stem cells obtained from the author's culture. The dried material was ground in a grinder 3 times for 1 min each time stopping the process after 15 s and the temperature was measured. If it increased >25°C, the process was stopped until cooling down. Dry material ground in such way was divided into two parts and the sonication process was carried out according to the parameters described above. The process was divided due to the type of sonotrode, which is not suitable for working with volumes above 800 mL of extract.

For each part, the following was used:

* Frequency no higher than 25 KHz, so as not to allow the transition of phytochemicals to their free radical form
* Amount of energy delivered: 40 W*s/mL, at a ratio of dry material: solvent 1:20
* Amplitude value of 40%, electronically adjustable
* Pulsating sonicator duty cycle $\alpha = 4/5$ $\alpha = \tau_{ON}/(\tau_{ON} + \tau_{OFF})$

$$\tau_{ON} = \text{sonicator generator on time} = 10 \text{ s}$$

$$\tau_{OFF} = \text{sonicator generator off time} = 2 \text{ s}$$

* Sonication power value 33-36 W.

After filtration three times to separate the plant residue, 994.5 g of filtrate was obtained, which was subjected to water evaporation to about 1/3 the amount of the flask contents. The distillation was stopped and the amount of solution remaining in the flask was weighed. An empty flask (298.90 g) was weighed at the beginning of the process. 399.096 g was left from which 5.7964 g was taken for metabolite content analysis.

Of this amount, after water evaporation at 80°C, 0.2808 g of metabolite dry matter remained, so the rotary evaporator flask contained 393.30 g of solution containing 19.052 g of metabolites. Maintaining the ratio of metabolites 22% to water 49.39% as shown in Table 1, 42.77 g of water + 19.052 g of metabolites or 61.822 g of solution must remain in the flask and therefore 331.478 g of water must be evaporated. After completing the vacuum distillation process, 62.32 g of solution was obtained, which was further filtered using Whatman No. 1 filter paper. A filtrate of 61.85 g was obtained, which contained 19.052 g of metabolites.

The obtained extract can then be encapsulated in liposomes.

Example 4. Testing of stem cell extracts obtained from selected plant species from *in vitro* **culture with potential use in cosmetic products in the context of assessing their safety of use and desired biological activity for active ingredients included in the composition**

I. Testing the biological activity of extracts obtained from the stem cells

1. Evaluation of antioxidant (anti-aging) properties by DPPH assay

1.1. DPPH Assay

The antioxidant activity of the extracts was tested using the DPPH (2,2-diphenyl-1-picrylhydrazyl) assay using 96-well plates. This method is based on the quenching reaction of a synthetic stable radical such as DPPH. An ethanolic solution of the DPPH reagent having a dark purple color under the influence of antioxidants present in the sample is reduced, with a change in color to yellow. The decrease in absorbance of the solution is proportional to the amount of the oxidized form of 1,1-diphenyl-2-picrylhydrazyl that remains in the solution.

A 0.5 mM methanolic solution of DPPH was used to perform the assay. Plant extracts (1, 2, 3, 4, 5) and the reference compound trolox which is a strong antioxidant were dissolved in dimethylsulfoxide (DMSO) to a concentration of 10 mg/mL. The extracts were shaken on a minishaker and heated at 70°C for 30 minutes, then filtered through a filter with a pore diameter of 45 $\mu$m. The final concentrations of the extracts were 0.1, 0.2, 0.4, 0.6, 0.8 and 1 mg/mL, the reference compound was tested at concentrations of 0.01, 0.02, 0.04, 0.06, 0.08 and 0.1 mg/mL. After adding equal volumes of DPPH and extract 1-5 solutions at different concentrations to the wells, the plate was incubated at 37°C for 30 min, and the absorbance was measured at 517 nm.

Fig. 1 shows the capacity of extracts 1-5 to scavenge free radicals in the DPPH assay. The assay results are expressed in trolox equivalents (TEs) (mg TE/g dry weight). This unit expresses the DPPH radical quenching capacity of 1 g dry weight of the tested extract equivalent to the DPPH radical quenching capacity of 1 mg of trolox. All assays were performed in triplicate, and the results were averaged.

Tested extracts 1-5 showed similar antioxidant activity. Extract 3 showed significantly lower (about two-times) antioxidant properties.

2. Evaluation of regenerative properties (proliferative activity) in crystal violet assay using the keratinocyte line (HaCat)

2.1 Crystal violet assay

Keratinocytes (RRID:CVCL_0038 line) were plated into a 96-well plate at a density of about $1*10^4$ cells per well. After 24 hours, the medium was replaced with one containing the appropriate compounds or without the test compounds (control). The cells were then incubated for 24 and 72 hours. After this time, the medium was removed from the wells and the cells were rinsed with PBS (without ions) warmed to about 37°C. The cells were fixed by adding 0.5 mL of 3.7% formaldehyde to each well. After 15 min, formaldehyde was replaced with 0.3 mL of crystal violet solution. Cells were stained for 10 min and then rinsed with water. The next step was to add 0.5 mL of decolorizer. After 30 minutes, absorbance was measured at 570 nm.

Fig. 2a shows the proliferation of keratinocytes of the HaCat line incubated in the presence of tested extracts 1-5 for 24 hours and Fig. 2b-for 72 hours. Cell proliferation was determined using crystal violet assay. Each experiment was performed in triplicate. The graphs show the number of live, proliferating cells expressed as a percentage of control (cells not incubated with the test compounds) $\pm$ SEM.

The tested extracts 1-5 stimulate keratinocyte proliferation in the concentration range of 1-50 $\mu$g/mL. The strongest effect was observed for extracts 2 and 3. At a concentration of 100 $\mu$g/mL, the obtained extracts inhibited keratinocyte proliferation.

II. Testing the safety of extracts obtained from stem cells

1. Exclusion of mutagenic/genotoxic effects in the Ames test

1.1. Ames test

The Ames test is a key tool in assessing the mutagenicity of substances *in vitro.* It is an essential part of genotoxicity testing and is required for preclinical safety assessments of biologically active ingredients. The test allows the identification of substances that cause mutations such as reading frame shifts or base pair substitutions. The test uses modified strains of *Salmonella typhimurium* bacteria carrying mutations in the operon of the gene encoding the amino acid histidine. The mutagenic potential of the compound is assessed by the number of bacterial revertants appearing, which, as a result of the mutation, have regained the ability to grow on histidine-free medium. The results are compared to positive controls, which are reference mutagens, and negative control, which determines the number of spontaneous mutations occurring in given strains.

The aim of the study was to assess the mutagenic potential of three extracts obtained from stem cells using two strains of *Salmonella typhimurium* carrying two types of mutations: TA 98—reading frame shift and TA 100—base pair substitution.

The experimental procedure was carried out basing on the manufacturer's experimental protocol (Xenometrix).

*Table 8: Assessment of mutagenic activity of selected extracts by Ames test.*

| Number of positive wells per plate | | | |
|---|---|---|---|
| | | *Salmonella typhimurium* | |
| Extract tested | Concentration (mg/mL) | TA98 | TA100 |
| | | MOI* | |
| PRLAB20021703 | 0.125 | 0.8 | 1.3 |
| | 0.25 | 0.8 | 1.0 |
| | 0.5 | 0.7 | 1.1 |
| | PC** | 5.6 | 3.0 |
| PRLAB20021702 | 0.125 | 0.7 | 0.9 |
| | 0.25 | 0.8 | 1.0 |
| | 0.5 | 0.5 | 0.9 |
| | PC** | 6.9 | 2.4 |
| PRLAB20021701 | 0.125 | 0.7 | 1.4 |
| | 0.25 | 0.9 | 1.4 |
| | 0.5 | 0.7 | 1.7 |
| | PC** | 5.6 | 3.0 |
| *MOI - Multiplicity of induction compared to baseline (baseline = average number of positive wells for zero-dose control + 1 SD); SD - standard deviation; a compound for which there is a reproducible increase of more than 2.0 or a clear dose-dependent response is referred to as a mutagen* | | | |
| **Positive controls (PCs): 2-nitrofluorene (2-NF) at a concentration of 2 μg/mL (TA98); 4-nitroquinoline N-oxide (4-NQO) at a concentration of 0.1 μg/mL (TA 100)* | | | |

For both strains of *Salmonella typhimurium* tested, no mutagenic/genotoxic effect of the tested extracts was observed.

2. Estimation of cytotoxic activity in MTT assay against normal skin cells (keratinocytes, HaCat)

2.1 MTT assay

The MTT assay is based on the ability of the mitochondrial dehydrogenase enzyme (present in living cells) to reduce the water-soluble tetrazolium salt (3-(4,5-dimethylthiazol-2-yl)-2,5-diphenyltetrazolium bromide) to insoluble formazan, which is the dark blue product of the above reaction. Dead, or metabolically inactive cells do not show the activity of the dehydrogenase enzyme and are unable to carry out the reaction. When the formazan crystals are dissolved in DMSO or isopropanol, a colored solution is formed, the color intensity of which is measured spectrophotometrically in the wavelength range of 492-570 nm. The amount of colored reduced MTT is proportional to the oxidative activity of the cell mitochondria, and under strictly defined experimental conditions to the number of metabolically active (living) cells in the population.

Keratinocytes (RRID:CVCL_0038 line) were plated at a density of $1 \times 10^4$ in 96-well plates. After overnight culture, cells were treated with increasing doses of compounds (1-100 μg/mL) and incubated for 24 hours. Vinblastine (100 μg/mL) was used as the reference compound. After the cells were exposed to the compounds for 24 hours, 10 μL of MTT reagent (Sigma Aldrich) was added to each well. After 4 hours of incubation (37°C, 5% $CO_2$), the medium was removed. To dissolve the formed formazan crystals, DMSO (dissolution solution) was added to each well. The optical density of the solution was then determined at 570 nm on a microplate reader (Spectra iD3 Max, Molecular Devices).

Fig. 3 shows the viability of keratinocytes of the HaCat line incubated in the presence of the tested extracts 1-5 for 24 hours. Cell viability was determined using MTT assay. Each experiment was performed in triplicate. The graphs show the number of viable cells expressed as a percentage of control (cells not treated with compounds) $\pm$ SEM.

The results clearly indicate that the tested extracts 1-5 in the concentration range of 1-50 $\mu$g/mL do not reduce the viability of keratinocytes.

3. Exclusion of hepatocytotoxic and neurocytotoxic effects in a cell-based model, HepG2 and SHSY-5Y, respectively, using MTT assay

Samples were coded according to the scheme proposed by the company (pdf document sent via email dated January 22, 2020).

3.1. MTT assay

The MTT assay is based on the ability of the mitochondrial dehydrogenase enzyme (present in living cells) to reduce the water-soluble tetrazolium salt (3-(4,5-dimethylthiazol-2-yl)-2,5-diphenyltetrazolium bromide) to insoluble formazan, which is the dark blue product of the above reaction. Dead, or metabolically inactive cells do not have the activity of the dehydrogenase enzyme and are unable to carry out the reaction. When the formazan crystals are dissolved in DMSO or isopropanol, a colored solution is produced, the color intensity of which is measured spectrophotometrically in the wavelength range of 492-570 nm. The amount of colored reduced MTT is proportional to the oxidative activity of the cell mitochondria, and under strictly defined experimental conditions to the number of metabolically active (living) cells in the population.

Cells of the HepG2 line (ATCC, HB-8065) were plated at a density of $1 \times 10^4$ in 96-well plates. After overnight culture, cells were treated with increasing doses of compounds (1-100 $\mu$g/mL) and incubated for 24 hours. Doxorubicin (100 $\mu$g/mL) was used as the reference compound. After the cells were exposed to the compounds for 24 hours, 10 $\mu$L of MTT reagent (Sigma Aldrich) was added to each well. After 4 hours of incubation (37°C, 5% $CO_2$), the medium was removed. To dissolve the formed formazan crystals, DMSO (dissolution solution) was added to each well. The optical density of the solution was then determined at 570 nm on a microplate reader (Spectra iD3 Max, Molecular Devices).

3.2. Hepatocytotoxic effects

Fig. 4 shows the viability of hepatocytes of the HepG2 line incubated in the presence of tested extracts 1-5 for 24 hours. Cell viability was determined using MTT assay. Each experiment was performed in triplicate. The graphs show the number of viable cells expressed as a percentage of control (cells not treated with compounds) $\pm$ SEM.

The results showed that extracts 1-5 did not affect the viability of HepG2 line cells in the concentration range of 1-100 $\mu$g/mL.

3.3. Neurocytotoxic effect

Fig. 5 shows the viability of SHSY-5Y line cells incubated in the presence of tested extracts 1-5 for 24 hours. Cell viability was determined using MTT assay. Each experiment was performed in triplicate. The graphs show the number of viable cells expressed as a percentage of control (cells not treated with compounds) $\pm$ SEM.

The tested extracts 1-5 do not affect the viability of the SHSY-5Y line cells in the concentration range of 1-100 $\mu$g/mL.

Conclusions:

1. The results confirm the safety of the tested extracts 1-5 against keratinocytes, with no hepatocytotoxic or neurocytotoxic effects.

2. No mutagenic/genotoxic effects were demonstrated for PRLAB20021703, PRLAB20021702, or PRLAB20021701 extracts.

3. Extracts 1-5 show moderate antioxidant (anti-aging) and proliferative (regenerative) activity. It seems that extract 2 in terms of biological activity is the most promising.

**Claims**

1. A method of preparing composition containing plant metabolite extract, which includes:

    - Grinding of plant material to obtain a particle size of not more than 0.5 mm, preferably not more than 0.4 mm, with grinding carried out at a temperature not higher than 35°C;
    - Maceration of the ground plant material with distilled water, wherein the weight ratio of plant material to distilled water is 1:20 per dry plant material;
    - Extraction of the plant material previously subjected to maceration using ultrasonic extraction and stirring, with cooling, wherein the weight ratio of plant material to distilled water is 1:20 per dry plant material;
    - Separation of the extract from the plant material using filtration, preferably vacuum filtration;
    - Concentration of the obtained plant metabolite extract, under strongly reduced pressure and at a temperature not higher than 40°C;
    - Adding and mixing of further components of the composition including water, glycerin, citric acid, sodium benzoate, gluconolactone, and calcium gluconate to obtain the finished composition containing plant metabolite extract.

2. The method according to claim 1, wherein fresh plant material, either dried at 40 °C in a dryer or lyophilized, is used.

3. The method according to claim 1 or 2, wherein the grinding is carried out in an electric grinder once or multiple times over a period of 1 second to 2 minutes.

4. The method according to claim 1 or 2 or 3, wherein the maceration is carried out over a period of 20 to 60 minutes, preferably 40 minutes, using a mechanical stirrer, at 200 to 600 rpm, preferably 450 rpm.

5. The method according to any of claims 1 to 4, wherein the shoots of *Kalanchoe pinnata* and *Calendula officinalis* containing stem cells are used as plant material.

6. The method according to any of claims 1 to 5, wherein cooling in the extraction process is carried out using ice water.

7. The method according to any of claims 1 to 6, wherein stirring in the extraction process is carried out using either a mechanical stirrer or a magnetic stirrer.

8. The method according to any of claims 1 to 7, wherein the mechanical stirrer is placed in the reaction vessel at an angle of 65°.

9. The method according to any of claims 1 to 8, wherein the following ultrasonication parameters are used in the extraction process:

    • Frequency not higher than 25 KHz,
    • Amount of energy delivered: 40 W*s/mL,
    • Amplitude value of 40%, electronically adjustable,
    • Pulsating sonicator duty cycle $\alpha = 4/5$ $\alpha = \tau_{ON}/(\tau_{ON} + \tau_{OFF})$

$$\tau_{ON} = \text{sonicator generator on time} = 10\ s$$

$$\tau_{OFF} = \text{sonicator generator off time} = 2\ s$$

    • Sonication power value 33-36 W,
    • Temperature below 30°C.

10. The method according to any of claims 1 to 9, wherein the concentration of the obtained plant metabolite extract is carried out in a vacuum rotary evaporator under a pressure of 30 to 60 mbar, preferably 40 to 55 mbar.

11. The method according to any of claims 1 to 10, wherein the other components of the composition are added in the following proportion by weight:

| Water | 49.99 |
|---|---|
| Glycerin | 26.20 |
| Plant stem cell extract | 22.00 |
| Citric acid | 0.60 |
| Sodium benzoate | 0.30 |
| Gluconolactone | 0.90 |
| Calcium gluconate | 0.01 |

12. Composition containing plant metabolite extract, **characterized in that** it consists of the components in the following weight ratio:

| Water | 49.99 |
|---|---|
| Glycerin | 26.20 |
| Plant stem cell extract | 22.00 |
| Citric acid | 0.60 |
| Sodium benzoate | 0.30 |
| Gluconolactone | 0.90 |
| Calcium gluconate | 0.01 |

13. The composition according to claim 12, **characterized in that** as the plant stem cells extract has the extract from the shoots of *Kalanchoe pinnata* and *Calendula officinalis* containing stem cells.

Fig. 1

Fig. 2a

Fig. 2b

Fig. 3

Fig. 4

# SHSY-5Y

Fig. 5

Europäisches Patentamt
European Patent Office
Office européen des brevets

# EUROPEAN SEARCH REPORT

Application Number

EP 22 46 1654

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2013/052244 A1 (WEINREB MARK [US] ET AL) 28 February 2013 (2013-02-28) * paragraph [0002]; claim 1 * | 1-13 | INV. A61K8/9789 A61Q19/08 A61K36/28 A61K36/41 |
| Y | CN 107 789 316 A (GUANGZHOU SALIAI STEMCELL SCIENCE & TECHNOLOGY CO LTD) 13 March 2018 (2018-03-13) * example 5 * | 1-13 | |
| Y | US 2020/276256 A1 (NOWAK SYLWIA [PL]) 3 September 2020 (2020-09-03) * paragraph [0009]; claim 1; figures 1-4 * | 1-13 | |
| Y | FR 3 000 390 A1 (CLARINS LAB [FR]) 4 July 2014 (2014-07-04) * claims 1,3, 7 * | 1-13 | |
| Y | US 2021/228662 A1 (RAHIMI MARYAM [US] ET AL) 29 July 2021 (2021-07-29) * paragraphs [0106], [0107] * | 1-13 | |
| Y | US 2018/110815 A1 (DRAKE KAREN [US]) 26 April 2018 (2018-04-26) * paragraph [0031]; claim 5 * | 1-13 | TECHNICAL FIELDS SEARCHED (IPC) A61K A61Q |
| A | Gulzar Alam ET AL: "WOUND HEALING POTENTIAL OF SOME MEDICINAL PLANTS", International Journal of Pharmaceutical Sciences Review and Research Page, 1 January 2011 (2011-01-01), XP055279419, Retrieved from the Internet: URL:http://globalresearchonline.net/journalcontents/volume9issue1/Article-026.pdf [retrieved on 2016-06-09] * table 1 * | 1-13 | |

The present search report has been drawn up for all claims

| Place of search | Date of completion of the search | Examiner |
|---|---|---|
| Munich | 16 June 2023 | Werner, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 46 1654

This annex lists the patent family members relating to the patent documents cited in the above-mentioned European search report.
The members are as contained in the European Patent Office EDP file on
The European Patent Office is in no way liable for these particulars which are merely given for the purpose of information.

16-06-2023

| Patent document cited in search report | | Publication date | Patent family member(s) | | Publication date |
|---|---|---|---|---|---|
| US 2013052244 | A1 | 28-02-2013 | US 2013052244 A1 | | 28-02-2013 |
| | | | WO 2013033365 A2 | | 07-03-2013 |
| CN 107789316 | A | 13-03-2018 | NONE | | |
| US 2020276256 | A1 | 03-09-2020 | CA 3061545 A1 | | 01-11-2018 |
| | | | CN 110831566 A | | 21-02-2020 |
| | | | EP 3614997 A1 | | 04-03-2020 |
| | | | US 2020276256 A1 | | 03-09-2020 |
| | | | WO 2018197668 A1 | | 01-11-2018 |
| FR 3000390 | A1 | 04-07-2014 | NONE | | |
| US 2021228662 | A1 | 29-07-2021 | CN 113993532 A | | 28-01-2022 |
| | | | EP 3890477 A1 | | 13-10-2021 |
| | | | EP 3911346 A1 | | 24-11-2021 |
| | | | US 2020179275 A1 | | 11-06-2020 |
| | | | US 2020179276 A1 | | 11-06-2020 |
| | | | US 2020179470 A1 | | 11-06-2020 |
| | | | US 2021228662 A1 | | 29-07-2021 |
| | | | WO 2020117309 A1 | | 11-06-2020 |
| | | | WO 2020117508 A1 | | 11-06-2020 |
| US 2018110815 | A1 | 26-04-2018 | US 2018110815 A1 | | 26-04-2018 |
| | | | WO 2018075086 A1 | | 26-04-2018 |

EPO FORM P0459

For more details about this annex : see Official Journal of the European Patent Office, No. 12/82